# EUROPEAN PATENT APPLICATION

(11) **EP 2 995 612 A1**
(43) Date of publication of application: **16.03.2016**
(21) Application number: 14306411.1
(22) Date of filing: 12.09.2014
(51) Int. Cl.: C07D 217/04, C07D 235/06, C07D 239/42, C07D 207/06, C07D 209/08, C07D 211/14, C07D 211/46, C07D 211/58, C07D 211/62, C07D 295/15, C07K 5/04, C07D 209/52

(54) **Novel non-natural amino acids, their process of preparation and uses thereof**

(71) Applicant: Université de Strasbourg, 67000 Strasbourg (FR); Centre National de la Recherche Scientifique (CNRS), 75016 Paris (FR)
(72) Inventor: Bihel, Frédéric, 67640 Fegersheim (FR); Schneider, Séverine, 67000 Strasbourg (FR); Ftouni, Hussein, 67400 Illkirch-Graffenstaden (FR); Schmitt, Martine, 67000 Strasbourg (FR)
(74) Representative: Blot, Philippe Robert Emile

(57) **Abstract**

The present invention concerns novel non-natural amino acids, more particularly non-natural analogs of arginine, ornithine or lysine, their process of preparation and their uses. In particular, such novel amino acids have the formula (I):

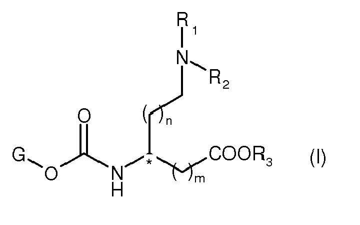

## Description

The present invention concerns novel non-natural amino acids, more particularly non-natural analogs of arginine, ornithine or lysine, their process of preparation and their uses in the preparation of peptides.

While peptides are the keystones of most of the vital processes, only few peptide-based drugs were marketed in the 20^{th} century. Among other criteria, low bioavailability and metabolic instability led to a global reject of peptides as drug candidates by the pharmaceutical industry.

However, in the last decade, new therapeutic strategies were promoted, and more than 100 peptide-based drugs have reached the market. In order to circumvent the inherent problems of bioavailability and stability of peptides, protein-like backbones called peptidomimetics were developed in order to mimic native peptides, but with better pharmacological properties. Their altered chemical structure allows them to adjust their molecular properties such as stability or biological activity, in particular to improve interactions between the peptide and its targeted protein.

One way of preparing peptidomimetics is by incorporating non-natural amino acids in their peptidic sequences. Non-natural amino acids can be generated from natural amino acids through chemical modifications of the backbone such as amine alkylation, backbone extension, cyclization or isosteric replacements. Another axis of interest is the design of "non-natural" side chains, which improve the interactions between the peptide and its targeted protein. Novel non-natural amino acids are thus of great interest for the development of peptidomimetics and for the development of new drugs.

A large diversity of non-natural amino acids were reported, especially non-natural amino acids bearing various aromatic side chains. In contrast, stable amino acids with side chains mimicking arginine, ornithine or lysine derivatives are still needed.

There is thus a need for novel non-natural amino acids, in particular for novel non-natural amino acids mimicking arginine, ornithine or lysine, useful in the preparation of peptides.

One aim of the present invention is to provide novel non-natural amino acids, useful in the preparation of peptides or polypeptides, and more particularly peptidomimetics. Another aim of the present invention is to provide a process of preparation of said amino acids. One aim of the present invention is to provide said non-natural amino acids with good yield and/or good selectivity, if possible with minimum purification steps.

One specific aim of the present invention is to provide said non-natural amino acids with retention of chirality during the preparation thereof.

Another aim of the present invention is to provide said non-natural amino acids at low costs.

Another aim of the present invention is to provide different types of non-natural amino acids.

One specific aim of the present invention is to provide access to gamma and delta amino acids exhibiting an amino group in gamma or delta position, respectively.

The present invention thus relates to an amino acid wherein the N-terminal end is protected by a protective group Gp of formula: 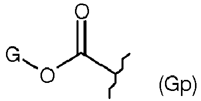 forming together with the nitrogen atom of said N-terminal end a carbamate group and wherein the G residue comprises at least one aromatic cyclic group optionally substituted, said amino acid further comprising a side-chain comprising a tertiary amine or a hydrazine.

Surprisingly, the inventors found that novel non-natural amino acids protected by a Gp group defined as above are stable and are especially useful in the preparation of peptides and/or polypeptides, preferably peptidomimetics. The Gp group may be cleaved *in vitro* and/or *in vivo* without affecting the rest of the amino acid structure and/or the peptide structure.

The amino acids of the invention may be used as analogs of arginine, ornithine or lysine and thus may be introduced in peptide sequences, to modulate the activity or the physical properties of peptides and/or polypeptides, leading to peptidomimetics. The amino acids of the invention may be directly incorporated in peptides and/or polypeptides by Solid or Liquid Phase Peptide Synthesis (SPPS or LPPS) processes. Moreover, said amino acids can be easily obtained at industrial-scale.

The present inventors discovered a process for the preparation of said amino acids, having a high selectivity for the reduction of the -C(O)-N-function present in the compounds of the invention, and having a high yield of amino acids. Indeed, it is difficult to perform selective reduction of the -C(O)-N-function in compounds of the invention especially due to the presence of a carbamate function and an ester group.

It has been discovered that reduction with Ru₃(CO)₁₂ enables obtaining the compounds of the invention with good yield and selectivity. Surprisingly, the present inventors were able to prepare amino acids using a reduction reaction selective toward the amide group, in the presence of a carbamate group and an ester group.

J. T. Reeves et al., (Adv. Synth. Catal. 2013, 355, 47-52) describe a procedure for the reduction of amides, using Ru₃(CO)₁₂ (triruthenium dodecacarbonyl) and TMDS (1,1,3,3-tetramethyldisiloxane). However, J.T. Reeves et al. do not disclose the reduction of a tertiary amide into a compound such as the amino acids of the invention, which cannot tolerate hard operating conditions. Said amino acids comprise an ester group or an acid carboxylic group, and a carbamate group, the latter being structurally close of an amide function. According to the inventors' knowledge, the selective reduction of amino acids of the invention has never been performed, and was unexpected.

More importantly, the process of preparation of the invention allows the retention of the chirality of said amino acids, which may be of importance regarding their biological activity.

Said process is advantageously low-cost and fast. In particular, the purification step may be simplified: purification by column chromatography may not be needed.

Lastly, said process leads to a high yield of amino acids according to the invention. In one embodiment, the yield is superior or equal to 78%, more particularly, superior or equal to 80%.

### Definitions

By "amino acid" it is understood an organic compound comprising an amine (-NH₂) functional group, herein called the N-terminal end, a carboxylic acid (-C(O)OH) functional group being optionally esterified by an alkyl group, herein called C-terminal end, and a side-chain.

The amino acids can be classified according to the position of the side-chain as follows:
- alpha- (α) amino acids when the amino group is attached to the carbon atom immediately adjacent to the carboxylic acid group,
- beta- (β-) amino acids when the carbon atom to which the amino group is attached, is separated from the carboxylic acid group by one other carbon atom,
- gamma- (γ-) amino acids when the carbon atom to which the amino group is attached, is separated from the carboxylic acid group by two other carbon atoms, or
- delta- (δ-) amino acids when the carbon atom to which the amino group is attached is separated from the carboxylic acid group by three other carbon atoms.

By "tertiary amine" is notably meant a functional group consisting of a nitrogen atom linked to three carbon atoms chosen among the group consisting of carbon atoms sp³, carbon atoms sp² and carbon atoms sp, wherein the double bond of said carbon atoms sp² is linked to another carbon atom or to a nitrogen atom, and wherein the triple bond of said carbon atoms sp is linked to another carbon atom.

By "carbamate" it is understood the functional group -N-C(O)O-.

In the context of the present invention, by "hydrazine" group is meant a functional group chosen from N-NH₂, N-NH(C₁-C₁₀)alkyl and N-N[(C₁-C₁₀)alkyl]₂,

In the context of the present invention, the term "alkyl" means a saturated aliphatic hydrocarbon group which may be linear, branched or cyclic. "Branched" means that one or lower alkyl groups such as methyl, ethyl or propyl are attached to a linear alkyl chain. Alkyl groups preferably comprise from 1 to 10 carbon atom(s). Preferred alkyl groups comprise 1, 2, 3, 4 or 5 carbon atom(s).

In the context of the present invention, "alkenyl" refers to an alkyl group as defined above, comprising at least one double bond.

In the context of the present invention, the term "alkylene" means a saturated aliphatic hydrocarbon divalent radical which may be linear or branched preferably comprising from 1 to 10 carbon atom(s). Preferred alkylene groups may have 1, 2 or 3 carbon atom(s).

In the context of the present invention, the term "heterocyclyl" refers to a saturated monocyclic or bicyclic hydrocarbon ring system, wherein any ring atom capable of substitution may be substituted by a substituent and wherein one or more carbon atom(s) are replaced by one or more heteroatom(s) such as nitrogen atom(s), oxygen atom(s) and sulfur atom(s); for example 1 or 2 nitrogen atom(s), 1 or 2 oxygen atom(s), 1 or 2 sulfur atom(s) or a combination of different heteroatoms, preferably 1 or 2 nitrogen atom(s).

For example, the heterocyclyl group is a pyrrolidine group, a piperidine group, a piperazine group, an azepan group or a 6-aza-bicyclo[3.2.1]octane group.

The term "aryl" refers to an aromatic monocyclic, bicyclic or tricyclic hydrocarbon ring system comprising for example from 6 to 14 carbon atoms wherein any ring atom capable of substitution may be substituted by a substituent.

In the context of the present invention, "aromatic cyclic group" refers to an aryl group as defined above. In a preferred embodiment, "aromatic cyclic group" refers to tricyclic hydrocarbon ring system. In one embodiment, preferred aryl groups are 9H-fluorene. In one embodiment, preferred aryl groups are phenyl.

In the context of the present invention, the term "heteroaryl" refers to an aryl as defined above wherein one or more carbon atom(s) are replaced by one or more heteroatom(s) such as nitrogen atom(s), oxygen atom(s) and sulfur atom(s); for example 1 or 2 nitrogen atom(s), 1 or 2 oxygen atom(s), 1 or 2 sulfur atom(s) or a combination of different heteroatoms, preferably 1 or 2 nitrogen atom(s). For example, the heteroaryl group is a pyrimidine group, a 1H-benzimidazole, a 1,2,3,4-tetrahydro-isoquinoline group or a 2.3-dihydro-1H-indole group.

The term "halogen" refers to the atoms of the group 17 of the periodic table and includes in particular fluorine, chlorine, bromine, and iodine atoms.

In the context of the present invention, by "alkyl groups comprising intra-chain functional groups" is meant an alkyl group as defined above wherein one or more carbon atom(s) are replaced by functional groups for example such as amide, ester, carboxy, and/or by heteroatoms such as nitrogen atom, oxygen atom or sulphur atom, and the like.

### Amino acids of the invention

The invention relates to an amino acid wherein the N-terminal end is protected by a protective group Gp of formula: 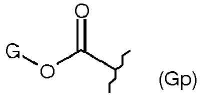 forming together with the nitrogen atom of said N-terminal end a carbamate group and wherein the G residue comprises at least one aromatic cyclic group optionally substituted, said amino acid further comprising a side-chain comprising a tertiary amine or a hydrazine.
For the sake of clarity, 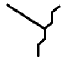 represents a covalent bond linked to said nitrogen atom of said N-terminal end.

The amino acids herein described may have asymmetric centers. Amino acids of the present invention containing an asymmetrically substituted atom may be isolated in optically active or racemic forms. It is well-known in the art how to prepare optically active forms, such as by resolution of racemic forms or by synthesis from optically active starting materials. All chiral, diastereomeric, racemic forms and all geometric isomeric forms of an amino acid are intended, unless the stereochemistry or the isomeric form is specifically indicated.

The term "pharmaceutically acceptable salt" refers to salts which retain the biological effectiveness and properties of the amino acids of the invention and which are not biologically or otherwise undesirable. Pharmaceutically acceptable acid addition salts may be prepared from inorganic and organic acids, while pharmaceutically acceptable base addition salts can be prepared from inorganic and organic bases. For a review of pharmaceutically acceptable salts see Berge, et al. ((1977) J. Pharm. Sd, vol. 66, 1). For example, the salts include those derived from inorganic acids such as hydrochloric, hydrobromic, sulfuric, sulfamic, phosphoric, nitric, and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicyclic, sulfanilic, fumaric, methanesulfonic, and toluenesulfonic acid and the like.

In one embodiment, the side-chain of said amino acid is a (C₁-C₃)alkyl, comprising a tertiary amine or a hydrazine group, preferably a tertiary amine. In particular, the side-chain of said amino acid does not comprise a N-C(O)O-tertbutyl group, which is neither a tertiary amine, nor a hydrazine group. In particular, the side-chain of said amino acid does not comprise a N-C(O)O-tertbutyl group and/or a N-C(O) group.

In one embodiment, the amino acid has formula (I): 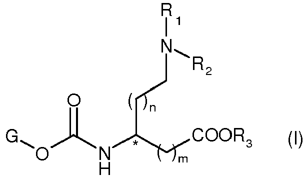 wherein:
- R₁ and R₂ are independently selected from the group consisting of:
   (C₁-C₁₀)alkyl, (C₆-C₁₀)aryl and (C₁-C₁₀)alkylene-(C₆-C₁₀)aryl;
   said alkyl, alkylene and aryl groups being optionally substituted by at least one substituent;
   or R₁ and R₂ may form together with the nitrogen atom carrying them a (5-10)membered heterocyclyl group or (6-10)membered heteroaryl group; said heterocyclyl and heteroaryl groups being optionally substituted by at least one substituent;
   or one of R₁ or R₂ is selected from the group consisting of:
      NH₂, NH(C₁-C₁₀)alkyl and N[(C₁-C₁₀)alkyl]₂, said alkyl groups being optionally substituted by at least one substituent, and the other one of R₁ or R₂ is selected from the group consisting of:
      (C₁-C₁₀)alkyl, (C₆-C₁₀)aryl and (C₁-C₁₀)alkylene-(C₆-C₁₀)aryl;
      said alkyl, alkylene and aryl groups being optionally substituted by at least one substituent,
      wherein R₁ and R₂ may form together with the nitrogen atom carrying them a (5-10)membered heterocyclyl group or (6-10)membered heteroaryl group; said heterocyclyl and heteroaryl groups being optionally substituted by at least one substituent;
- R₃ is selected from the group consisting of: H and (C₁-C₁₀)alkyl, alkyl groups being optionally substituted by at least one substituent;
- G is a group comprising at least one aromatic cyclic group;
- n is 0,1 or 2; and
- m is 0, 1 or 2;
or its pharmaceutically acceptable salts, hydrates or hydrated salts or its polymorphic crystalline structures, racemates, diastereomers or enantiomers.

In one embodiment, said compound is a hydrochloride salt of a compound of formula (I). In one embodiment, the compound of formula (I) has a L or a D configuration: 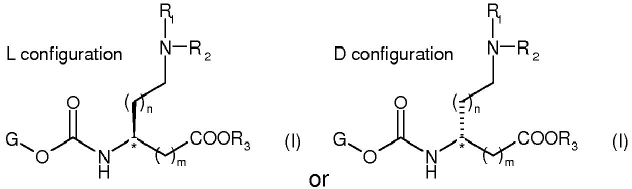 Preferably, the compound of formula (I) has a L configuration.

In a particular embodiment, G is: 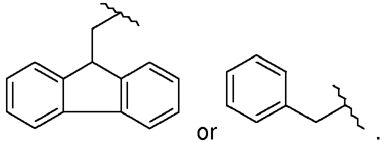 More particularly, G is: 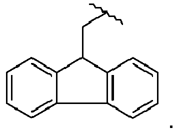 In one embodiment, "Fmoc" refers to the following group:

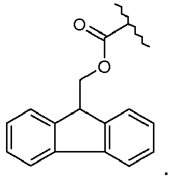

In one embodiment R₃ is H, methyl, ethyl, isopropyl or tert-butyl, preferably H or tert-butyl. More particularly, R₃ is H.

When m is 0, said amino acid is an alpha amino acid. In one embodiment, n is 2 and m is 0. In another embodiment, n is 2, m is 0 and R₃ is H. In a particular embodiment, when m is 0, n is 1 or 2. In another embodiment, when n is 0, m is 1 or 2.

In one embodiment, R₁ and R₂ are independently selected from the group consisting of:
(C₁-C₆)alkyl, (C₆-C₁₀)aryl and (C₁-C₆)alkylene-(C₆-C₁₀)aryl; said alkyl and aryl groups being optionally substituted by one or more substituent(s), preferably one substituent, selected from the group consisting of:
   CN, OH, (C₁-C₁₀)alkyl and C(O)O(C₁-C₁₀)alkyl, more particularly CN;
   or R₁ and R₂ may form together with the nitrogen atom carrying them a (5-10)membered heterocyclyl group or (6-10)membered heteroaryl group;
   said heterocyclyl and heteroaryl groups being optionally substituted by one or more substituents selected from the group consisting of:
      CN, OH, (C₁-C₁₀)alkyl, C(O)O(C₁-C₁₀)alkyl, (5-6)membered heterocyclyl, (5-6)membered heteroaryl group and (C₁-C₄)alkylene-phenyl;
      or one of R₁ or R₂ is NH₂; and the other one of R₁ or R₂ is selected from the group consisting of: (C₁-C₆)alkyl, (C₆-C₁₀)aryl and (C₁-C₆)alkylene-(C₆-C₁₀)aryl.

In another embodiment, R₁ and R₂ are independently chosen from the group consisting of: (C₁-C₄)alkyl, phenyl and (C₁-C₄)alkylene-phenyl; said alkyl group being possibly substituted by a CN group;
or R₁ and R₂ may form together with the nitrogen atom carrying them a pyrrolidine, a piperidine, an azepane, a piperazine, a 6-azabicyclo[3.2.1]octane, a 1 H-benzimidazole, a 2,3-dihydro-1H-indole or a tetrahydroisoquinoline group;
said rings being possibly substituted by one or two substituent(s) independently chosen from: (C₁-C₄)alkyl, OH, C(O)O(C₁-C₄)alkyl, a piperidine, a pyrimidine group and (C₁-C₄)alkylene-phenyl;
or one of R₁ or R₂ is NH₂; and the other one of R₁ or R₂ is (C₁-C₄)alkylene-phenyl.

In one embodiment, said amino acid is an alpha amino acid, of formula a): 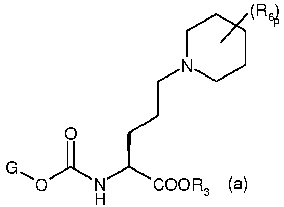 wherein:
- p is 0,1, 2, 3, 4 or 5;
- R₆ is a substituent, for example independently selected from the group consisting of: CN, OH, (C₁-C₁₀)alkyl, C(O)O(C₁-C₁₀)alkyl, (5-10)membered heterocyclyl, (6-10)membered heteroaryl group and (C₁-C₁₀)alkylene-(C₆-C₁₀)aryl;
- G and R₃ being defined above.

In one embodiment, R₆ is chosen from OH, (C₁-C₄)alkyl, C(O)O(C₁-C₄)alkyl, (5-6)membered heterocyclyl, (6)membered heteroaryl group and (C₁-C₄)alkylene-phenyl. "Independently selected" means that when p is 2 to 5, R₆ can be different at each occurrence. In one embodiment, p is 1 and R₆ is in the para position.

In one embodiment, G, R₁, R₂ and R₃ are substituted by at least one substituent, preferably by one or two substituent(s), independently selected from the group consisting of:
halogen, CN, OH, NH₂, NO₂, CHO, (C₁-C₁₀)alkyl, (C₂-C₁₀)alkenyl, -O-(C₁-C₁₀)alkyl, C(O)R, NHR, NRR' and C(O)OR; R and R' being independently from each other selected from (C₁-C₁₀)alkyl groups; wherein alkyl groups optionally bear one or more intra-chain functional groups and
when R₁ and R₂ form together with the nitrogen atom carrying them a (5-10)membered heterocyclyl group or (6-10)membered heteroaryl group, said heterocyclyl and heteroaryl group are optionally substituted by at least one substituent independently selected from the group consisting of:
   halogen, CN, OH, NH₂, NO₂, CHO, (C₁-C₁₀)alkyl, (C₂-C₁₀)alkenyl, -O-(C₁-C₁₀)alkyl, C(O)R, NHR, NRR', C(O)OR, (5-10)membered heterocyclyl, (6-10)membered heteroaryl and (C₁-C₁₀)alkylene-(C₆-C₁₀)aryl; R and R' being independently from each other selected from (C₁-C₁₀)alkyl groups; wherein alkyl groups optionally bear one or more intra-chain functional groups.

In one embodiment, R₁ and R₂ are independently selected from the group consisting of:
(C₁-C₁₀)alkyl, (C₆-C₁₀)aryl and (C₁-C₁₀)alkylene-(C₆-C₁₀)aryl;said alkyl, alkylene and aryl groups being optionally substituted by one or two substituent(s), independently selected from the group consisting of:
   halogen, CN, OH, NH₂, NO₂, CHO, (C₁-C₁₀)alkyl, (C₂-C₁₀)alkenyl, -O-(C₁-C₁₀)alkyl, C(O)R, NHR, NRR' and C(O)OR; R and R' being independently from each other selected from (C₁-C₁₀)alkyl groups; wherein alkyl groups optionally bear one or more intra-chain functional groups or
   when R₁ and R₂ form together with the nitrogen atom carrying them a (5-10)membered heterocyclyl group or (6-10)membered heteroaryl group, said heterocyclyl and heteroaryl group are optionally substituted by one or two substituent(s) independently selected from the group consisting of:
      halogen, CN, OH, NH₂, NO₂, CHO, (C₁-C₁₀)alkyl, (C₂-C₁₀)alkenyl, -O-(C₁-C₁₀)alkyl, C(O)R, NHR, NRR', C(O)NHR, C(O)NRR', C(O)OR, (5-10)membered heterocyclyl, (6-10)membered heteroaryl and (C₁-C₁₀)alkylene-(C₆-C₁₀)aryl;R and R' being independently from each other selected from (C₁-C₁₀)alkyl groups; wherein alkyl groups optionally bear one or more intra-chain functional group;
      or one of R₁ or R₂ is selected from the group consisting of:
         NH₂, NH(C₁-C₁₀)alkyl and N[(C₁-C₁₀)alkylh, and the other one of R₁ or R₂ is selected from the group consisting of: (C₁-C₁₀)alkyl, (C₆-C₁₀)aryl and (C₁-C₁₀)alkylene-(C₆-C₁₀)aryl;
         said alkyl, alkylene and aryl groups being optionally substituted by one or two substituent(s), independently selected from the group consisting of:
            halogen, CN, OH, NH₂, NO₂, CHO, (C₁-C₁₀)alkyl, (C₂-C₁₀)alkenyl, -O-(C₁-C₁₀)alkyl, C(O)R, NHR, NRR', C(O)NHR, C(O)NRR' and C(O)OR; R and R' being independently from each other selected from (C₁-C₁₀)alkyl groups; wherein alkyl groups optionally bear one or more intra-chain functional groups.

Particular embodiments and/or specific features can be combined with each other.

The present invention also relates to the following specific compounds:

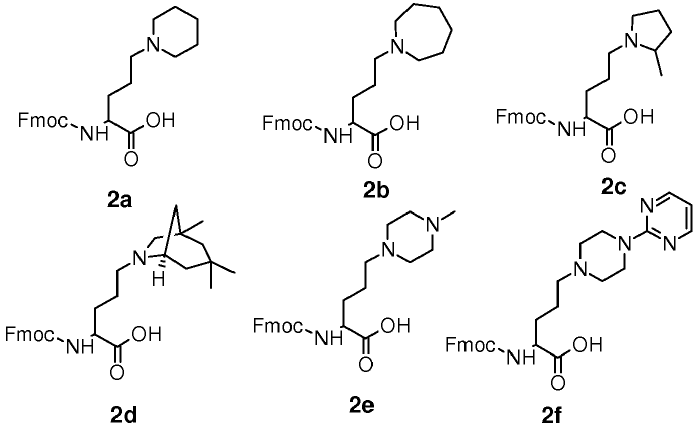

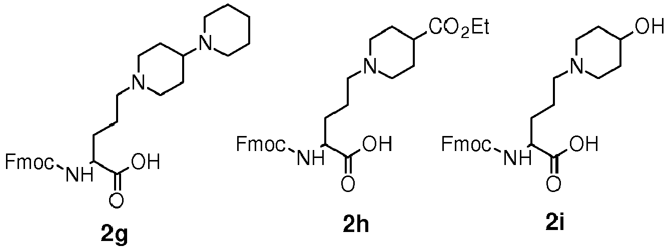

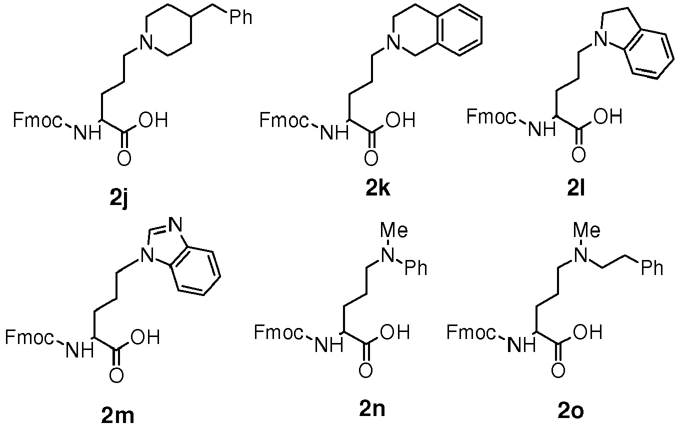

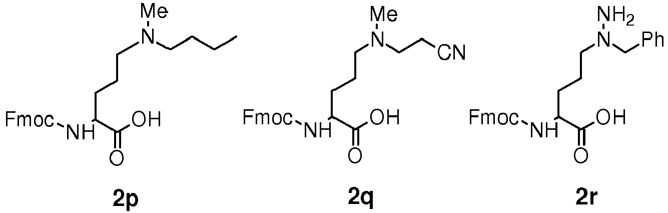

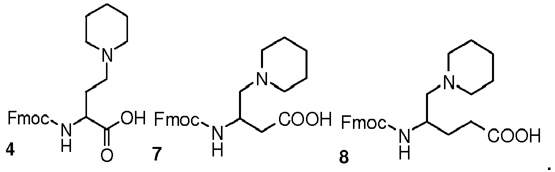

In one embodiment, the present invention relates to the following specific compounds:

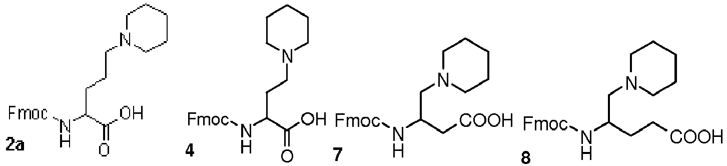

In one embodiment, the present invention relates to the following specific compounds:

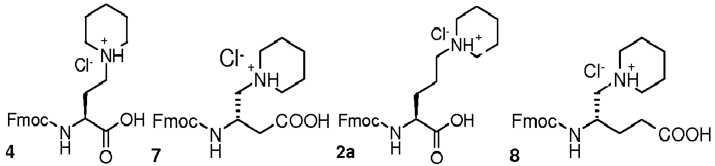

The present invention also relates to the use of the amino acid defined according to the invention, for the preparation of a peptide and/or a polypeptide, preferably by liquid or solid phase.

In one embodiment, said peptide and/or polypeptide is a peptidomimetic.
The preparation process of said peptide and/or polypeptide by liquid or solid phase is well-known in the art. It is described for example in Amblard, M.; Fehrentz, J. A.; Martinez, J.; Subra, G., Methods and Protocols of modern solid phase peptide synthesis. Mol Biotechnol 2006, 33, 239-254.

The present invention also relates to a method for preparing compounds comprising at least one deprotected amino acid defined according to the invention. In one embodiment, the invention relates to the preparation of a peptide and/or a polypeptide comprising at least one deprotected amino acid defined according to the invention.

Such method for preparing a compound comprising at least one amino acid, comprises the following steps:
i) preparing at least one amino acid as defined in any one of claims 1 to 10,
ii) deprotecting said amino acid,
iii) reacting the deprotected amino acid with a carboxylic acid using a coupling reagent such as benzotriazol-1-yl-oxytripyrrolidino phosphonium hexafluorophosphate (PyBOP) or N-[(dimethylamino)-1 H-1,2,3-triazolo[4,5-b] pyridin-1-yl-methylene]-N-methyl-methanaminium hexafluorophosphate N-oxide (HATU),
iv) obtaining a compound comprising at least one amino acid.

"Deprotected amino acid defined according to the invention" means an amino acid defined according to the invention which does not comprise G group. Amino acids defined according to the invention are deprotected according to known conditions. Such conditions are for example piperidine (20% in DMF), or diazabicyclo[5.4.0]undec-7-ene (DBU) in DMF.

### Process of preparation of said amino acids

Another object of the invention is a process for the preparation of an amino acid according to the invention, comprising the reaction of a compound of formula (II): 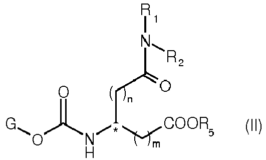 with Ru₃(CO)₁₂, in the presence of a solvent, preferably THF (tetrahydrofurane), to obtain a compound of formula (III): 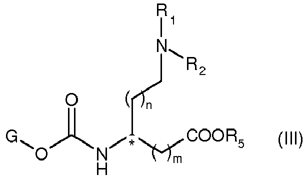 wherein G, R₁, R₂, m and n are defined as above and R₅ is a protective group, preferably a tert-butyl group.
In one embodiment, at least 3 mol% of Ru₃(CO)₁₂ is used in the reaction. In one embodiment, said reaction is performed with 5 mol% of Ru₃(CO)₁₂.

In one embodiment, said reaction is performed in THF. In another embodiment, said reaction is performed in THF, with a silane or siloxane, preferably TMDS (1,1,3,3-tetramethyldisiloxane). In another embodiment, at least 4 eq of TMDS is used in the reaction.

In one embodiment, the reaction temperature is between 15 and 70°C, advantageously between 15 and 50°C. For example the temperature is between 30 and 50°C.

In one embodiment, said reaction is performed with 3% to 5% of Ru₃(CO)₁₂, 4 eq to 8 eq of TMDS in THF. In one embodiment, said reaction is performed with 5 mol% of Ru₃(CO)₁₂, 8 eq of TMDS in THF, preferably at 40°C. In one embodiment, said reaction is performed with 3% of Ru₃(CO)₁₂, 6 eq of TMDS in THF, preferably at 40°C.

In one embodiment, said process further comprises the following steps:
a) purifying said compound of formula (I): 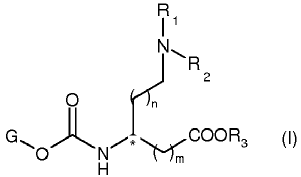 by adding trifluoroacetic acid, while evaporating the solvent, preferably THF, to obtain a compound of formula (IV): 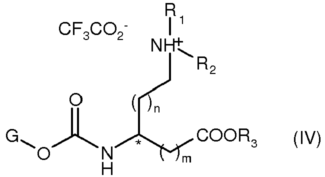 wherein G, R₁, R₂, R₃, m and n are defined above;
b) obtaining a salt or a hydrated salt of the compound of formula (I), preferably by adding an acid, preferably a strong acid, for example in ether, and
c) optionally neutralizing the salt to obtain a compound of formula (I), its hydrates, its polymorphe crystalline structures, racemates, diastereoisomers or enantiomers.

Strong acids are well-known in the art. A strong acid is one that completely ionizes in a solution. In water, one mole of a strong acid dissolves yielding one mole of H⁺ (as hydronium ion H₃O⁺) and one mole of the conjugate base. Among strong acids, hydrochloric acid (HCl), hydroiodic acid (HI), hydrobromic acid (HBr) and sulfuric acid (H₂SO₄) can be cited. In one embodiment, the acid is HCl.

The following examples show that the process of the invention has selectivity toward the amide reduction and allows the retention of chirality of the amino acids according to the invention. The obtained amino acids are stable and useful in the preparation of peptidomimetics. Some preparative examples are also given below, without limitation of the present invention.

In the following examples, the temperature is room temperature (20°C), unless contrarily indicated and the pressure is atmospheric pressure (101 325 Pa) unless contrarily indicated.

### EXAMPLES

In the following examples DIEA (Diisopropylethylamine),DCM (dichloromethane),HATU (N-[(dimethylamino)-1H-1,2,3-triazolo[4,5-b]pyridin-1-yl-methylene]-N-methyl-methanaminium hexafluorophosphate N-oxide), TMDS (Tetramethyldisiloxane) and TFA (trifluoroacetic acid) are used.

### Example 1: Preparation of compound 2a

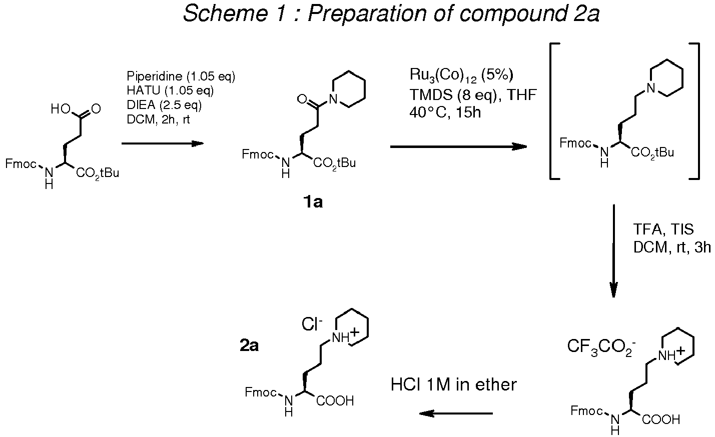

To a solution of commercially available Fmoc-Glu(OH)-OtBu (Fluorochem, CAS N° : 84793-07-7) (2g, 4.70 mmol, 1 equiv.) in DMF (18 mL) were added DIEA (Fischer Scientific, CAS N° : 7087-68-5) (1.23 mL, 7.05 mmol 1.5 equiv.), piperidine (Alfa Aesar, CAS N°: 110-89-4) (4.93 mmol, 1.05 equiv.), and finally HATU (1-[Bis(dimethylamino)methylene]-1 H-1,2,3-triazolo[4,5-b]pyridinium 3-oxid hexafluorophosphate (ChemImpex, CAS N°: 148893-10-1), 1.93g, 4.93 mmol, 1.05 equiv.). The reaction mixture was stirred at room temperature for 3 hours, and the volatiles were removed under reduced pressure. The resulting mixture was solubilized in EtOAc, and treated with aqueous 1 N HCl, aqueous saturated NaHCO₃, and aqueous saturated NaCl. Layers were separated, and the organic phase was dried (MgSO₄), filtered, and concentrated to afford compound 1a.

Tetramethyldisiloxane (Alfa Aesar, CAS N°: 3277-267) (6.5 mL, 35.7 mmol, 8 equiv.) was added to dodecakis(methanidyldyneoxidanium)triruthenium (Aldrich, CAS N°:15243-33-1) (0.144 g, 0.223 mmol, 0.05 equiv.) under argon. Mixture was stirred for 5 minutes, then a solution of compound **1a** (4.46 mmol, 1 equiv.) in 24 mL of THF was added, and the reaction mixture was stirred for 16 hours under argon at 40°C. Volatiles were removed in vacuo. Reaction mixture was diluted with DCM (24 mL), and cooled to 0°C in an ice bath. Trifluoroacetic acid (20 mL) was slowly added at 0°C. After addition, the reaction mixture was stirred at room temperature for 4 hours. Volatiles were removed under reduced pressure, and the resulting mixture was diluted in diethyl ether (10 mL). A solution of HCl (2M) in diethyl ether (48 mL) was slowly added to the mixture, and the reaction mixture was stirred at room temperature for 5 minutes, affording a white powder after filtration. Compound 2a was obtained after evaporation and lyophilization.

### Example 2: Comparison of different catalysts

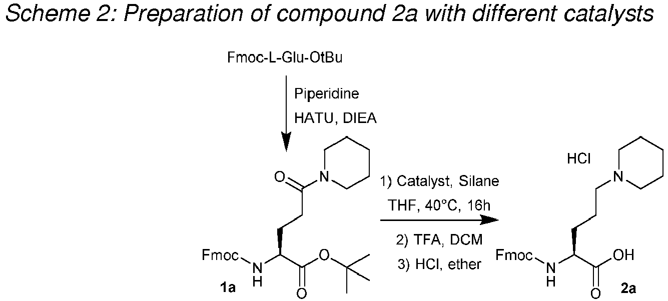

Starting from the commercially available Fmoc-L-Glu-OtBu (Fluorochem, CAS N° : 84793-07-7), of formula: 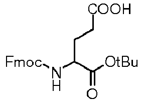 piperidine was coupled on the side chain to obtain compound **1a.**

Two catalytic systems were evaluated (see Table 1), involving Zn,^{10g} and Ru,^{10q} for reduction of the carboxamide function in a selective manner, as compared to both carbamate (Fmoc) and tertbutylique ester functions. THF was used as solvent.

It has to be noted that using Zn(OAc)₂ as a catalyst with (EtO)₃SiH according to the reaction described in Beller et al. (see Das, S.; Addis, D.; Zhou, S.; Junge, K.; Beller, M., Zinc-Catalyzed Reduction of Amides: Unprecedented Selectivity and Functional Group Tolerance. Journal of the American Chemical Society 2010, 132 (6), 1770-1771) does not lead to the reduction obtained according to the invention. Indeed, zinc-mediated reduction was not observed (entry 1), while Ru₃(CO)₁₂ in combination with Et₂SiH₂ led to good yield and Ru₃(CO)₁₂ in combination with TMDS led to compound **2a** in high yields (superior or equal to 78%). The reduction of the glutamine derivatives 1 is performed preferably with at least 3 mol% of catalyst (Table 1, compare entries 7 & 8).

Easy to handle, the reaction can be done under air conditions. After the completion of the reduction step, the resulting terbutyl ester was recovered in poor yields (<30%), after column chromatography using normal or reverse phases. Treatment of this terbutyl ester with trifluoroacetic acid led to compound **2a** as a TFA salt. Precipitation in ether of this salt led to medium yields (≈50%). Then, trifluoroacetate counter-ion was exchanged with HCl 1M in diethylether, affording a white precipitate **2a** in 81% molar yield.

**Table 1: Yields obtained with different catalysts**

| **Entry** | **Catalyst** | **Mol**% | **Silane** | **Equiv.** | **Yield^{b} (%)** |
|---|---|---|---|---|---|
| 1 | Zn(OAc)₂ | 3 | (EtO)₃SiH | 8 | 0 |
| 4 | Ru₃(CO)₁₂ | 3 | Et₂SiH₂ | 8 | 74 |
| 5 | Ru₃(CO)₁₂ | 3 | (EtO)₃SiH | 8 | 60 |
| 6 | Ru₃(CO)₁₂ | 3 | iPr₃SiH | 8 | 0 |
| 7 | Ru₃(CO)₁₂ | 3 | TMDS | 8 | 80 |
| 9 | Ru₃(CO)₁₂ | 5 | TMDS | 8 | 80 |
| 11 | Ru₃(CO)₁₂ | 3 | TMDS | 4 | 78 |
| 12 | Ru₃(CO)₁₂ | 3 | TMDS | 6 | 81 |

| | | | | | |
|---|---|---|---|---|---|
| ^{a} Conditions: A mixture of catalyst, silane, 1a (0.2 mmol) in THF was stirred at 40°C for 16h. After reaction completion, volatiles were removed and a mixture of TFA in DCM (1:1, v:v) was added. After 3h, the mixture was concentrated, then precipitated in HCl (1 M) in ether. ^{b} Determined by HPLC using caffeine as external. | | | | | |

### Example 3: Gram-scale reaction

The scope of the reaction was extended to the gram-scale with a large set of tertiary amines (Scheme 4). Under these conditions, compounds **2** were globally obtained in excellent yields after precipitation in ether. The reaction was compatible with hindered amide **(2d)** and allowed to introduce a second chiral center **(2c, 2d).** Several functional groups were tolerated, such as tertiary amines **(2e, 2g),** aromatic guanidine **(2f),** ester **(2h),** and alcohol **(2i). A** series of amines bearing an aromatic cycle was also easily synthesized (**2j**-**o**). While aniline derivatives were well tolerated, acyclic amines **(2n-q)** were also easily obtained in high yields. Interestingly, after coupling the Bn-NH-NH-Boc on Fmoc-L-Glu-OtBu, the hydrazide function was reduced to afford the corresponding hydrazine derivative **2r** as a non-natural analog of lysine.

Starting from Fmoc-L-Asp-OtBu, compound **4** was obtained. Starting from commercially available Fmoc-L-Asp(OtBu)-OH or Fmoc-L-Glu(OtBu)-OH, piperidine was coupled to the C-terminus part to afford compounds **5** and **6.** Ruthenium-based reduction of the resulting carboxamide led to the gamma-amino acid 6 in excellent yields, as shown in Scheme 3 below.

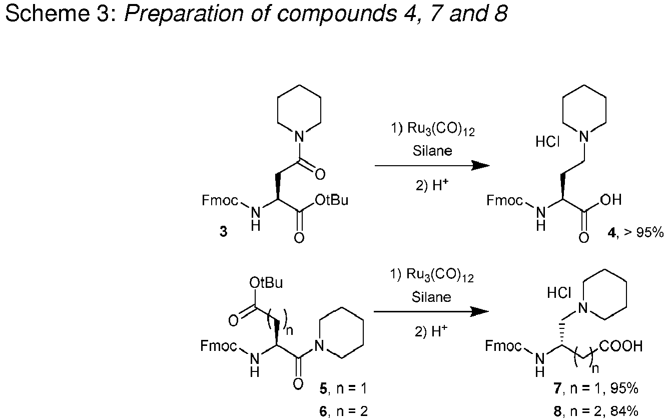

The absence of purification on column allowed to easily produce large quantity of non-natural amino acids, with 98% minimum purity measured by HPLC and NMR. However, both analytical technics did not allow to detect ruthenium. Using ICP-mass spectrometry, 2.1% of ruthenium in compound 2a was quantified.

### Scheme 4: Obtained yields of the amino acids of the invention

Conditions: A mixture of catalyst (5 mol%), silane (8 eq.), 1a (1 g, 2 mmol) in THF was stirred at 40°C for 16h. After reaction completon, volatiles were removed and a mixture of TFA in DCM (1:1, v:v) was added. After 3h, the mixture was concentrated, then precipitated in HCl (1M) in ether. ^{b} Purified by flash chromatography on reverse phase.

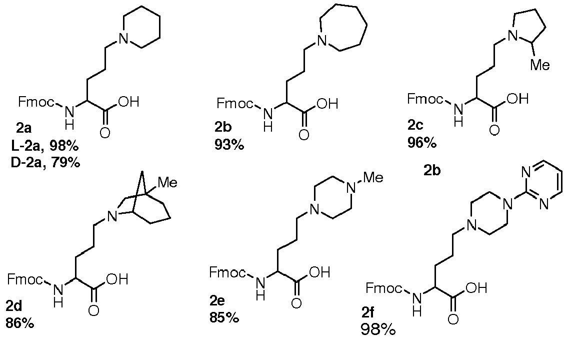

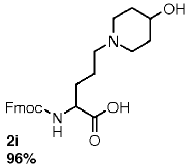

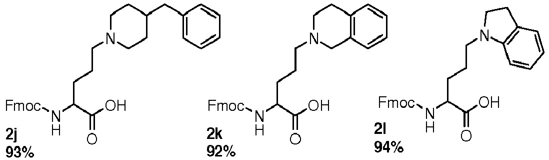

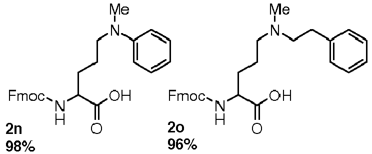

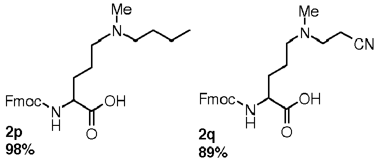

### Example 4: Retention of chirality

The question as to if the process according to the invention may induce a racemization of the Cα of the formed amino acids was evaluated.

To answer this question, both enantiomers **L-2a** and **D-2a** were coupled to H-L-Phe-NH₂ (Bachem, CAS N°: 5241-58-7) using HATU (ChemImpex, CAS N°: 148893-10-1) as coupling reagent. By HPLC, the racemization ratio as inferior to 1% was evaluated (data not shown). By repeating this last coupling reaction, it was determined that the resulting traces of racemization were occurring to the coupling reaction with H-Phe-NH₂, and not during the reductive process. Thus, the process according to the invention allows the retention of the chirality of the amino acids of the invention.

The analytical data for compounds 2a to 8 were as follows:

### Compound L-2a

### (S)-2-((((9H-fluoren-9-yl)methoxy)carbonyl)amino)-5-(piperidin-1-yl)pentanoic acid hydrochloride

¹H NMR (DMSO-d6, 400 MHz) : δ=7.90 (d, *J* = 7.5 Hz, 2H ), 7.72 (m, 2H ), 7.42 (dd, *J* = 7.5, 7.5 Hz, 2H), 7.33 (dd, *J* = 7.5, 7.5 Hz, 2H), 4.34 - 4.21 (m, 3H), 3.97 (td, *J* = 3.1, 8.6 Hz, 1 H), 3.35 (m, 2H), 2.97 (m, 2H), 2.80 (m, 2H), 1.83 - 1.61 (m, 9H), 1.44 - 1.30 (m, 1 H); ¹³C NMR (DMSO-d6, 100 MHz) : δ = 173.9, 156.7, 144.3, 141.2, 128.1, 127.6, 125.7, 120.6, 66.1, 55.8, 53.8, 52.6, 52.3, 47.1, 28.5, 22.8, 21.8, 20.6; HRMS (ESI+) calcd. for C₂₅H₃₀N₂O₄ [M + H]+ 423.2278, found 423.2278

### Compound D-2a

### (R)-2-((((9H-fluoren-9-yl)methoxy)carbonyl)amino)-5-(piperidin-1-yl)pentanoic acid hydrochloride

¹H NMR (DMSO-d6, 400 MHz) : δ=7.90 (d, *J* = 7.5 Hz, 2H ), 7.72 (m, 2H ), 7.42 (dd, *J* = 7.5, 7.5 Hz, 2H), 7.33 (dd, *J* =7.5, 7.5 Hz, 2H), 4.34 - 4.21 (m, 3H), 3.97 (td, *J* = 3.1, 8.6 Hz, 1 H), 3.35 (m, 2H), 2.97 (m, 2H), 2.80 (m, 2H), 1.83 - 1.61 (m, 9H), 1.44 - 1.30 (m, 1H); ¹³C NMR (DMSO-d6, 100 MHz) : δ = 173.9, 156.7, 144.3, 141.2, 128.1, 127.6, 125.7, 120.6, 66.1, 55.8, 53.8, 52.6, 52.3, 47.1, 28.5, 22.8, 21.8, 20.6; HRMS (ESI+) calcd. for C₂₅H₃₀N₂O₄ [M + H]+ 423.2278, found 423.2278

### Compound 2b

### (S)-2-((((9H-fluoren-9-yl)methoxy)carbonyl)amino)-5-(azepan-1-yl)pentanoic acid hydrochloride

¹H NMR (DMSO-d6, 400 MHz) : δ = 7.90 (d, *J* = 7.5 Hz, 2H ), 7.73 (d, *J* = 7.5 Hz, 1H ), 7.71 (d, *J =* 7.5 Hz, 1H ), 7.42 (dd, *J* = 7.5, 7.5 Hz, 2H), 7.34 (dd, *J* = 7.5, 7.5 Hz, 2H), 4.34 - 4.21 (m, 3H), 2.45 - 2.32 (m, 1 H), 3.25 - 3.12 (m, 2H), 3.12 - 2.94 (m, 4H), 1.93 - 1.70 (m, 6H), 1.69 - 1.50 (m, 6H); ¹³C NMR (DMSO-d6, 100 MHz) : δ = 173.3, 156.1, 143.7, 140.6, 127.6, 127.0, 125.2, 120.1, 65.6, 55.6, 53.3, 46.6, 27.9, 25.9, 22.7, 20.4; HRMS (ESI+) calcd. for C₂₆H₃₂N₂O₄ [M + H]⁺ 437.2435, found 437.2438

### Compound 2c

### (S)-2-((((9H-fluoren-9-yl)methoxy)carbonyl)amino)-5-((R)-2-methylpyrrolidin-1-yl)pentanoic acid hydrochloride

¹H NMR (DMSO-d6, 400 MHz) : δ = 7.89 (d, *J* = 7.5 Hz, 2H ), 7.73 (m, 2H ), 7.42 (dd, *J =* 7.5, 7.5 Hz, 2H), 7.34 (dd, *J* = 7.5, 7.5 Hz, 2H), 4.29 (m, 2H), 4.24 (m, 1 H), 3.98 (m, 1 H), 3.54 (m, 1 H), 3.23 (m, 1 H), 2.98 (m, 1 H), 2.87 (m, 1 H), 2.14 (m, 1 H), 1.90 (m, 3H), 1.81 - 1.55 (m, 5H); ¹³C NMR (DMSO-d6, 100 MHz) : δ = 173.4, 156.2, 143.8, 140.6, 127.6, 127.1, 125.2, 120.1, 65.7, 63.4, 53.3, 52.2, 51.3, 46.6, 30.8, 27.9, 21.6, 20.8, 15.1; HRMS (ESI+) calcd. for C₂₅H₃₀N₂O₄ [M + H]⁺ 423.2278, found 423.2271

### Compound 2d

### (S)-2-((((9H-fluoren-9-yl)methoxy)carbonyl)amino)-5-((1S,5R)-1-methyl-6-azabicyclo[3.2.1]octan-6-yl)pentanoic acid hydrochloride

¹H NMR (MeOD-d4, 400 MHz) : δ = 7.80 (d, *J =* 7.5 Hz, 2H), 7.67 (m, 2H), 7.40 (dd, *J* = 7.5 Hz, 2H), 7.32 (dd, *J* = 7.5 Hz, 2H), 4.41 (m, 1 H), 4.32 (m, 1 H), 4.23 (m, 2H), 3.90 (m, 1 H), 3.72 (dd, *J* = 48.4, 12.4 Hz, 1H), 3.20 (m, 2H), 2.88 - 2.73 (m, 1 H), 2.08 - 1.52 (m, 10H), 1.22 - 0.96 (m, 9H); HRMS (ESI+) calcd. for C₃₀H₃₈N₂O₄ [M + H]+ 491.2904, found 437.2895

### Compound 2e

### (S)-2-((((9H-fluoren-9-yl)methoxy)carbonyl)amino)-5-(4-methylpiperazin-1-yl)pentanoic acid hydrochloride

¹H NMR (DMSO-d6, 400 MHz) : δ = 8.52 (s, 1 H), 7.89 (d, *J=* 7.5 Hz, 2H), 7.73 (d, *J=* 7.5 Hz, 1H ), 7.71 (d, *J=* 7.5 Hz, 1H), 7.42 (dd, *J* = 7.5, 7.5 Hz, 2H), 7.34 (dd, *J* = 7.5, 7.5 Hz, 2H), 4.34 - 4.19 (m, 3H), 3.96 (m, 1 H), 3.75 - 3.59 (m, 4H), 3.54 - 3.30 (m, 4H), 3.24 - 3.05 (m, 2H), 2.81 (s, 3H), 1.95 - 1.60 (m, 4H); ¹³C NMR (DMSO-d6, 100 MHz) : δ = 173.3, 156.1, 143.7, 140.6, 127.6, 127.1, 125.2, 120.1, 65.6, 53.4, 51.2, 49.5, 47.9, 46.6, 27.8, 20.1; HRMS (ESI+) calcd. for C₂₅H₃₁N₃O₄ [M + H]+ 438.2387, found 438.2395

### Compound 2f

### (S)-2-((((9H-fluoren-9-yl)methoxy)carbonyl)amino)-5-(4-(pyrimidin-2-yl)piperazin-1-yl)pentanoic acid hydrochloride

¹H NMR (MeOD-d4, 400 MHz) : δ = 8.54 (d, *J* = 5.2 Hz, 2H), 7.80 (d, *J* = 7.6 Hz, 2H), 7.68 (d, *J =* 7.6 Hz, 1 H), 7.66 (d, *J =* 7.6 Hz, 1 H), 7.40 (dd, *J* = 7.6 Hz, 2H), 7.35 - 7.29 (m, 2H), 6.93 (dd, *J* = 5.2 Hz, 1 H), 4.84 (m, 2H), 4.41 (m, 1 H), 4.34 (m, 1 H), 4.23 (m, 2H), 3.76 - 3.63 (m, 2H), 3.63 - 3.46 (m, 2H), 3.28 - 3.10 (m, 4H), 2.02 - 1.70 (m, 4H); ¹³C NMR (MeOD-d4, 100 MHz) : δ = 175.1, 158.9, 158.6, 145.4, 142.7, 128.9, 128.3, 126.3, 121.1, 112.6, 68.2, 57.7, 54.7, 52.5, 42.7, 29.8, 21.9; HRMS (ESI+) calcd. for C₂₈H₃₁N₅O₄ [M + H]+ 502.2449, found 502.2458

### Compound 2g

### (S)-2-((((9H-fluoren-9-yl)methoxy)carbonyl)amino)-5-([1,4'-bipiperidin]-1'-yl)pentanoic acid hydrochloride

¹H NMR (DMSO-d6, 400 MHz) : δ = 7.89 (d, *J=* 7.5 Hz, 2H), 7.73 (d, *J=* 7.5 Hz, 1 H), 7.73 (d, *J* = 7.5 Hz, 1 H), 7.42 (dd, *J* = 7.5 Hz, 2H), 7.33 (dd, *J* = 7.5 Hz, 2H), 4.33 - 4.18 (m, 3H), 3.95 (m, 1 H), 2.99 (m, 5H), 2.53 (m, 4H), 2.20 (m, 2H), 1.89 - 2.05 (m, 2H), 1.78 - 1.43 (m, 12H); ¹³C NMR (DMSO-d6, 100 MHz) : δ = 173.7, 156.1, 143.7, 140.1, 127.6, 127.0, 125.2, 120.1, 65.5, 55.9, 53.6, 51.2, 49.1, 46.6, 28.5, 25.1, 23.3, 22.1, 22.0; HRMS (ESI+) calcd. for C₃₀H₃₉N₃O₄ [M + H]+ 506.3013, found 506.3010

### Compound 2h

### (S)-2-((((9H-fluoren-9-yl)methoxy)carbonyl)amino)-5-(4-(ethoxycarbonyl)piperidin-1-yl)pentanoic acid hydrochloride

¹H NMR (MeOD-d4, 400 MHz): δ = 7.78 (d, *J=* 7.5 Hz, 2H), 7.66 (d, *J=* 7.5 Hz, 1 H), 7.64 (d, *J=* 7.5 Hz, 1 H), 7.38 (dd, *J* = 7.5 Hz, 2H), 7.30 (dd, *J* = 7.5 Hz, 2H), 4.36 (m, 2H), 4.19 (t, *J* = 6.6 Hz, 1 H), 4.11 (q, *J=* 7.1 Hz, 2H), 4.01 (m, 1 H), 3.56 - 3.33 (m, 2H), 3.08 - 2.76 (m, 4H), 2.60 (m, 1 H), 2.16 - 2.04 (m, 2H), 2.03 - 1.87 (m, 2H), 1.83 - 1.45 (m, 4H), 1.21 (t, *J* = 7.1 Hz, 3H); ¹³C NMR (MeOD-d4, 100 MHz) : δ = 178.4, 174.7, 158.1, 145.6, 142.7, 128.9, 128.3, 126.3, 121.1, 67.8, 62.1, 57.8, 56.8, 52.3, 48.6, 31.1, 26.5, 21.5, 14.6; HRMS (ESI+) calcd. for C₂₈H₃₄N₂O₆ [M + H]+ 495.2490, found 495.2502

### Compound 2i

### (S)-2-((((9H-fluoren-9-yl)methoxy)carbonyl)amino)-5-(4-hydroxypiperidin-1-yl)pentanoic acid hydrochloride

¹H NMR (MeOD-d4, 400 MHz) : δ=7.79 (d, *J=* 7.5 Hz, 2H), 7.66 (d, *J=* 7.5 Hz, 1 H), 7.64 (d, *J* = 7.5 Hz, 1 H), 7.38 (dd, *J* = 7.5 Hz, 2H), 7.30 (dd, J= 7.5 Hz, 2H), 4.37 (m, 2H), 4.20 (t, *J* = 6.6 Hz, 1 H), 4.01 (m, 1 H), 3.89 (m, 1 H), 3.40 - 3.21 (m, 2H), 3.18 - 2.79 (m, 4H), 2.01 (m, 2H), 1.91 - 1.59 (m, 6H); ¹³C NMR (MeOD-d4, 100 MHz) : δ = 178.4, 158.1, 145.5, 142.7, 128.9, 128.3, 126.3, 121.1, 67.7, 57.7, 56.9, 48.6, 31.9, 31.2, 21.6; HRMS (ESI+) calcd. for C₂₅H₃₀N₂O₅ [M + H]+ 439.2227, found 439.2236

### Compound 2j

### (S)-2-((((9H-fluoren-9-yl)methoxy)carbonyl)amino)-5-(4-benzylpiperidin-1-yl)pentanoic acid hydrochloride

¹H NMR (MeOD-d4, 400 MHz): δ = 7.78 (d, *J=* 7.5 Hz, 2H), 7.67 (d, *J=* 7.5 Hz, 1 H), 7.65 (d, *J =* 7.5 Hz, 1 H), 7.38 (dd, *J* = 7.5 Hz, 2H), 7.34 - 7.24 (m, 4H), 7.22 - 7.13 (m, 3H), 4.40 (dd, *J* = 10.8, 6.5 Hz, 1 H), 4.31 (m, 1 H), 4.21 (m, 2H), 3.48 (d, *J* = 11.4 Hz, 2H), 3.07 (m, 2H), 2.84 (m, 2H), 2.58 (d, *J =* 6.5 Hz, 2H), 1.96 - 1.72 (m, 7H), 1.50 (m, 2H); ¹³C NMR (MeOD-d4, 100 MHz) : δ = 175.0, 158.8, 145.4, 142.7, 140.5, 130.3, 129.6, 128.9, 128.3, 127.5, 126.3, 121.1, 68, 1, 57.7, 54.6, 54.3, 48.4, 43.1, 36.8, 30.6, 29.8, 21.9; HRMS (ESI+) calcd. for C₃₂H₃₆N₂O₄ [M + H]+ 513.2748, found 513.2749

### Compound 2k

### (S)-2-((((9H-fluoren-9-yl)methoxy)carbonyl)amino)-5-(3,4-dihydroisoquinolin-2(1H)-yl)pentanoic acid hydrochloride

¹H NMR (DMSO-d6, 400 MHz) : δ=7.90 (d, *J=* 7.5 Hz, 2H), 7.74 (d, *J=* 7.5 Hz, 1 H), 7.73 (d, *J =* 7.5 Hz, 1 H), 7.42 (dd, *J* = 7.5 Hz, 2H), 7.34 (dd, *J* = 7.5 Hz, 2H), 7.30 - 7.21 (m, 3H), 7.18 (m, 1 H), 4.48 (m, 1 H), 4.38 - 4.19 (m, 4H), 4.01 (m, 1 H), 3.64 (m, 1 H), 3.34 - 3.13 (m, 4H), 3.01 (m, 1 H), 1.94 - 1.63 (m, 4H); ¹³C NMR (MeOD-d4, 100 MHz) : δ = 173.6, 156.4, 144.0, 141.0, 131.7, 128.8, 127.9, 127.4, 126.8, 125.5, 120.4, 65.9, 54.8, 53.6, 51.9, 48.9, 46.9, 28.2, 25.0, 20.7; HRMS (ESI+) calcd. for C₂₉H₃₀N₂O₄ [M + H]+ 471.2278, found 471.2293

### Compound 2I

### (S)-2-((((9H-fluoren-9-yl)methoxy)carbonyl)amino)-5-(indolin-1-yl)pentanoic acid hydrochloride

¹H NMR (MeOD-d4, 400 MHz) : δ=7.76 (d, *J=* 7.5 Hz, 2H), 7.64 (d, *J=* 7.5 Hz, 1 H), 7.61 (d, *J* = 7.5 Hz, 1 H), 7.52 - 7.40 (m, 4H), 7.34 (dd, *J* = 7.5 Hz, 2H), 7.26 (dd, *J* = 7.5 Hz, 2H), 4.32 (m, 2H), 4.18 (m, 2H), 3.91 (m, 2H), 3.56 (m, 2H), 2.17 - 1.67 (m, 6H); ¹³ C NMR (DMSO-d6, 100 MHz) : δ = 173.5, 156.0, 143.6, 140.5, 127.5, 126.9, 125.1, 119.9, 65.5, 53.3, 52.4, 46.5, 28.0, 27.7, 22.1; HRMS (ESI+) calcd. for C₂₈H₂₈N₂O₄ [M + H]+ 457.2122, found 457.2136

### Compound 2n

### (S)-2-((((9H-fluoren-9-yl)methoxy)carbonyl)amino)-5-(methyl(phenyl)amino)pentanoic acid hydrochloride

¹H NMR (MeOD-d4, 400 MHz) : δ = 7.72 (d, *J* = 7.5 Hz, 1H), 7.71 (d, *J* = 7.5 Hz, 1 H), 7.59 (dd, *J* = 7.5 Hz, 2H), 7.31 (dd, *J* = 7.5 Hz, 2H), 7.19 - 7.25 (m, 2H), 7.11 (dd, *J* = 7.5 Hz, 2H), 6.69 (d, *J =* 8.0 Hz, 2H), 6.59 (dd, *J* = 7.5 Hz, 1 H), 4.30 (m, 2H), 4.13 (m, 2H), 3.27 (m, 2H), 2.83 (s, 3H), 1.82 (m, 1 H), 1.60 (m, 3H); ¹³C NMR (MeOD-d4, 100 MHz) : δ = 176.0, 158.7, 150.7, 145.3, 142.7, 130.2, 128.9, 128.3, 126.3, 121.0, 117.9, 114.1, 68.0, 55.3, 53.5, 48.6, 38.9, 30.4, 24.1; HRMS (ESI+) calcd. for C₂₇H₂₈N₂O₄ [M + H]+ 445.2122, found 445.2126

### Compound 2o

### (S)-2-((((9H-fluoren-9-yl)methoxy)carbonyl)amino)-5-(methyl(phenethyl)amino)pentanoic acid hydrochloride

¹H NMR (MeOD-d4, 400 MHz) : δ = 7.79 (d, *J* = 7.5 Hz, 2H), 7.67 (d, *J* = 7.5 Hz, 1 H), 7.65 (d, *J* = 7.5 Hz, 1 H), 7.39 (dd, *J* = 7.5 Hz, 2H), 7.35 - 7.23 (m, 7H), 4.42 (dd, *J =* 10.1, 6.6 Hz, 1 H), 4.33 (m, 1 H), 4.22 (m, 2H), 3.38 (m, 2H), 3.22 (m, 2H), 3.06 (m, 2H), 2.91 (s, 3H), 1.96 (m, 1 H), 1.90 - 1.70 (m, 3H); ¹³ C NMR (DMSO-d6, 100 MHz) : δ = 173.9, 156.7, 144.2, 141.2, 137.5, 129.2, 129.1, 128.1, 127.6, 127.3, 125.7, 120.6, 66.2, 56.3, 54.8, 53.9, 47.1, 29.8, 28.3, 20.6; HRMS (ESI+) calcd. for C₂₉H₃₂N₂O₄ [M + H]+ 473.2435, found 473.2448

### Compound 2p

### (S)-2-((((9H-fluoren-9-yl)methoxy)carbonyl)amino)-5-(butyl(methyl)amino)pentanoic acid hydrochloride

¹H NMR (MeOD-d4, 400 MHz) : δ = 7.79 (d, *J=* 7.5 Hz, 2H), 7.68 (d, *J=* 7.5 Hz, 1 H), 7.66 (d, *J=* 7.5 Hz, 1 H), 7.39 (dd, *J* = 7.5 Hz, 2H), 7.31 (dd, *J* = 7.5 Hz, 2H), 4.41 (dd, *J* = 10.3, 6.7 Hz, 1 H), 4.33 (m, 1 H), 4.22 (m, 2H), 3.22 - 3.00 (m, 4H), 2.82 (s, 3H), 2.12 - 1.59 (m, 6H), 1.38 (m, 2H), 0.97 (t, *J* = 7.4 Hz, 1 H); ¹³C NMR (MeOD-d4, 100 MHz) : δ = 175.1, 158.9, 145.4, 142.7, 128.9, 128.3, 126.3, 121.1, 68.2, 57.5, 56.8, 54.7, 40.8, 29.8, 27.3, 22.0, 20.9, 14.0; HRMS (ESI+) calcd. for C₂₅H₃₂N₂O₄ [M + H]+ 425.2435, found 425.2453

### Compound 2q

### (S)-2-((((9H-fluoren-9-yl)methoxy)carbonyl)amino)-5-((2-cyanoethyl)(methyl)amino)pentanoic acid hydrochloride

¹H NMR (MeOD-d4, 400 MHz) : δ = 7.79 (d, *J* = 7.5 Hz, 2H), 7.66 (d, *J* = 7.5 Hz, 1 H), 7.65 (d, *J=* 7.5 Hz, 1 H), 7.38 (dd, *J* = 7.5 Hz, 2H), 7.30 (dd, *J* = 7.5 Hz, 2H), 4.38 (m, 2H), 4.21 (t, *J=* 6.7 Hz, 1 H), 4.07 (m, 1 H), 3.07 (t, *J* = 7.0 Hz, 2H), 2.80 (m, 4H), 2.54 (s, 3H), 1.90 - 1.54 (m, 4H); ¹³C NMR (MeOD-d4, 100 MHz) : δ = 177.5, 158.5, 145.5, 142.7, 128.9, 128.3, 126.3, 121.1, 119.1, 67.9, 57.5, 56.1, 53.0, 48.6, 41.0, 30.8, 23.1, 15.0; HRMS (ESI+) calcd. for C₂₄H₂₇N₃O₄ [M + H]+ 422.2074, found 422.2080

### Compound 2r

### (S)-2-((((9H-fluoren-9-yl)methoxy)carbonyl)amino)-5-(1-benzylhydrazinyl)pentanoic acid hydrochloride

¹H NMR (MeOD-d4, 400 MHz) : δ = 7.77 (d, *J* = 7.5 Hz, 2H), 7.66 (d, *J* = 7.5 Hz, 1 H), 7.64 (d, *J =* 7.5 Hz, 1 H), 7.48 - 7.32 (m, 7H), 7.28 (dd, *J* = 7.5 Hz, 2H), 4.33 (m, 2H), 4.24 - 4.04 (m, 4H), 3.04 (m, 2H), 1.93 (m, 1H), 1.77 (m, 3H); ¹³C NMR (MeOD-d4, 100 MHz) : δ = 175.4, 158.8, 145.2, 142.7, 131.4, 130.2, 128.9, 128.3, 126.3, 121.1, 68.1, 62.4, 57.1, 54.9, 48.5, 29.9, 23.0; MS (ESI+) calcd. for C₂₇H₂₉N₃O₄ [M + H]+ 460.2, found 460.2

### Compound 4

### (S)-2-((((9H-fluoren-9-yl)methoxy)carbonyl)amino)-4-(piperidin-1-yl)butanoic acid hydrochloride

¹H NMR (MeOD-d4, 400 MHz) : δ = 7.79 (d, *J* = 7.5 Hz, 2H), 7.67 (d, *J* = 7.5 Hz, 1 H), 7.65 (d, *J* = 7.5 Hz, 1 H), 7.38 (dd, *J* = 7.5 Hz, 2H), 7.31 (dd, *J* = 7.5 Hz, 2H), 4.46 - 4.32 (m, 2H), 4.27 (m, 1 H), 4.22 (t, *J =* 6.8 Hz, 1 H), 3.50 (m, 2H), 3.19 (m, 1 H), 3.12 (m, 1 H), 2.91 (q, *J* = 12.2 Hz, 2H), 2.35 (m, 1 H), 2.15 (m, 1 H), 1.90 (m, 2H), 1.80 (m, 3H), 1.50 (m, 1 H); ¹³C NMR (MeOD-d4, 100 MHz) : δ = 174.0, 158.8, 145.4, 142.7, 128.9, 128.3, 126.3, 121.1, 68.2, 55.5, 54.8, 54.5, 53.1, 27.4, 24.4, 22.7; HRMS (ESI+) calcd. for C₂₄H₂₈N₂O₄ [M + H]+ 409.2122, found 409.2121

### Compound 7

### (S)-3-((((9H-fluoren-9-yl)methoxy)carbonyl)amino)-4-(piperidin-1-yl)butanoic acid hydrochloride

¹H NMR (DMSO-d6, 400 MHz) : δ = 7.94 (d, *J* = 7.5 Hz, 2H ), 7.74 (dd, *J* = 7.5, 7.5 Hz, 2H), 7.69 (d, *J =* 8.2 Hz, 1 H), 7.47 (dd, *J* = 7.5, 7.5 Hz, 2H), 7.37 (m, 2H), 4.42 (m, 1 H), 4.31 (m, 3H), 3.45 (m, 2H), 3.21 (m, 2H), 2.96 (m, 2H), 2.73 (m, 2H), 2.01 - 1.62 (m, 5H), 1.42 (m, 1H); ¹³C NMR (DMSO-d6, 100 MHz) : δ = 171.4,155.5, 143.7, 140.7, 127.6, 127.0, 125.1, 120.1, 65.6, 58.4, 52.9, 51.9, 46.6, 43.6, 37.4, 21.9, 21.1; HRMS (ESI+) calcd. for C₂₄H₂₈N₂O₄ [M + H]+ 409.2122, found 409.2123

### Compound 8

### (S)-4-((((9H-fluoren-9-yl)methoxy)carbonyl)amino)-5-(piperidin-1-yl)pentanoic acid hydrochloride

¹H NMR (DMSO-d6, 400 MHz) : δ = 7.89 (d, *J* = 7.5 Hz, 2H ), 7.70 (d, *J* = 7.5 Hz, 2H ), 7.47 (d, *J=* 8.7 Hz, 1H ), 7.41 (dd, *J* = 7.5, 7.5 Hz, 2H ), 7.33 (dd, *J* = 7.5, 7.5 Hz, 2H ), 4.45 (dd, *J* = 6.8, 10.4 Hz, 1 H), 4.31 (m, 1 H), 4.22 (t, *J* = 6.5 Hz, 1 H), 3.89 (m, 1 H), 3.37 (m, 2H), 3.10 - 2.92 (m, 2H), 2.86 (m, 2H), 2.21 (m, 2H), 1.77 - 1.46 (m, 7H), 1.35 (m, 1H); ¹³C NMR (DMSO-d6, 100 MHz) : δ=173.8, 155.8, 143.7, 140.7, 127.6, 127.0, 125.2, 120.1, 65.4, 58.7, 52.6, 51.9, 46.7, 45.7, 29.7, 28.1, 21.9, 21.21; HRMS (ESI+) calcd. for C₂₅H₃₀N₂O₄ [M + H]+ 423.2278, found 423.2280

### Example 5: Use of the amino acids according to the invention

The utility of novel non-natural analogs of ornithine was demonstrated in a synthesis of compound 9 (Scheme 5), which is known for its ability to prevent opioid-induced hyperalgesia after oral administration in rodents. Starting from compound 2a, compound **9** was easily synthesized in a gram scale using standard peptide synthesis.

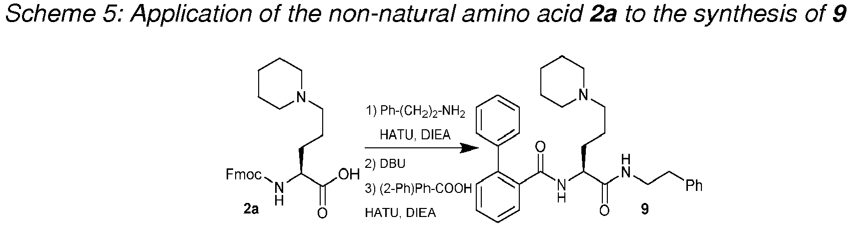

### Example 6: Stability of the amino acids of the invention

The amino acid **2a** was stored during 10 weeks at a temperature of -18°C without light exposure, no degradation was observed in HPLC.

## Claims

1. An amino acid wherein the N-terminal end is protected by a protective group Gp of formula: 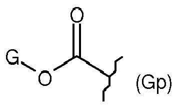 forming together with the nitrogen atom of said N-terminal end a carbamate group and wherein the G residue comprises at least one aromatic cyclic group optionally substituted, said amino acid further comprising a side-chain comprising a tertiary amine or a hydrazine.

2. The amino acid according to claim 1, having formula (I): 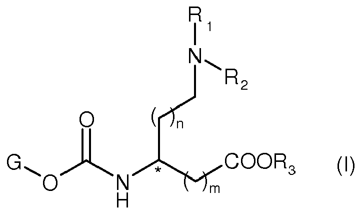 wherein:
- R₁ and R₂ are independently selected from the group consisting of:
(C₁-C₁₀)alkyl, (C₆-C₁₀)aryl and (C₁-C₁₀)alkylene-(C₆-C₁₀)aryl;
said alkyl, alkylene and aryl groups being optionally substituted by at least one substituent;
or R₁ and R₂ may form together with the nitrogen atom carrying them a (5-10)membered heterocyclyl group or (6-10)membered heteroaryl group; said heterocyclyl and heteroaryl groups being optionally substituted by at least one substituent;
or one of R₁ or R₂ is selected from the group consisting of:
NH₂, NH(C₁-C₁₀)alkyl and N[(C₁-C₁₀)alkyl]₂, said alkyl groups being optionally substituted by at least one substituent, and the other one of R₁ or R₂ is selected from the group consisting of:
(C₁-C₁₀)alkyl, (C₆-C₁₀)aryl and (C₁-C₁₀)alkylene-(C₆-C₁₀)aryl;
said alkyl, alkylene and aryl groups being optionally substituted by at least one substituent,
wherein R₁ and R₂ may form together with the nitrogen atom carrying them a (5-10)membered heterocyclyl group or (6-10)membered heteroaryl group; said heterocyclyl and heteroaryl groups being optionally substituted by at least one substituent;
- R₃ is selected from the group consisting of: H and (C₁-C₁₀)alkyl, alkyl groups being optionally substituted by at least one substituent;
- G is a group comprising at least one aromatic cyclic group;
- n is 0, 1 or 2; and
- m is 0, 1 or 2;
or its pharmaceutically acceptable salts, hydrates or hydrated salts or its polymorphic crystalline structures, racemates, diastereomers or enantiomers.

3. The amino acid of claim 1 or 2, wherein G is:

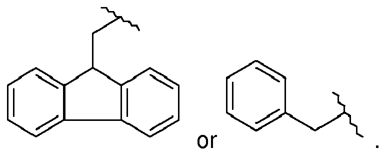

4. The amino acid of any one of claims 1 to 3, wherein G is:

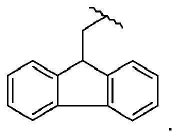

5. The amino acid of any one of claims 1 to 4, wherein R₃ is H, methyl, ethyl, isopropyl or tert-butyl.

6. The amino acid of any one of claims 1 to 5, wherein n is 2 and m is 0.

7. The amino acid of any one of claims 1 to 6, wherein R₁ and R₂ are independently selected from the group consisting of:
(C1-C6)alkyl, (C6-C10)aryl and (C1-C6)alkylene-(C6-C10)aryl; said alkyl and aryl groups being optionally substituted by one or more substituents selected from the group consisting of:
CN, OH, (C1-C10)alkyl and C(O)O(C1-C10)alkyl;
or R1 and R2 may form together with the nitrogen atom carrying them a (5-10)membered heterocyclyl group or (6-10)membered heteroaryl group;
said heterocyclyl and heteroaryl groups being optionally substituted by one or more substituents selected from the group consisting of:
CN, OH, (C1-C10)alkyl, C(O)O(C1-C10)alkyl, (5-6)membered heterocyclyl, (5-6)membered heteroaryl group and (C1-C4)alkylene-phenyl;
or one of R1 or R2 is NH2; and the other one of R1 or R2 is selected from the group consisting of:
(C1-C6)alkyl, (C6-C10)aryl and (C1-C6)alkylene-(C6-C10)aryl.

8. The amino acid of any one of claims 1 to 7, having a L configuration: 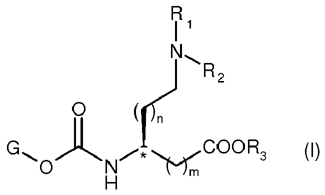 wherein G, R₁, R₂, R₃, m and n are defined in any one of claims 1 to 7.

9. The amino acid of claim 1, having formula (a): 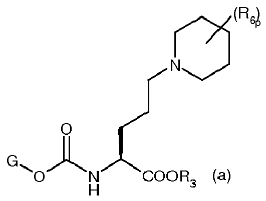 wherein:
- p is 0, 1, 2, 3, 4 or 5;
- R₆ is a substituent, for example independently selected from the group consisting of: CN, OH, (C₁-C₁₀)alkyl, C(O)O(C₁-C₁₀)alkyl, (5-10)membered heterocyclyl, (6-10)membered heteroaryl group and (C₁-C₁₀)alkylene-(C₆-C₁₀)aryl;
- G and R₃ being defined according to any one of claims 1 to 7.

10. The amino acid of any one of claims 1 to 9, having one of the following formulae:

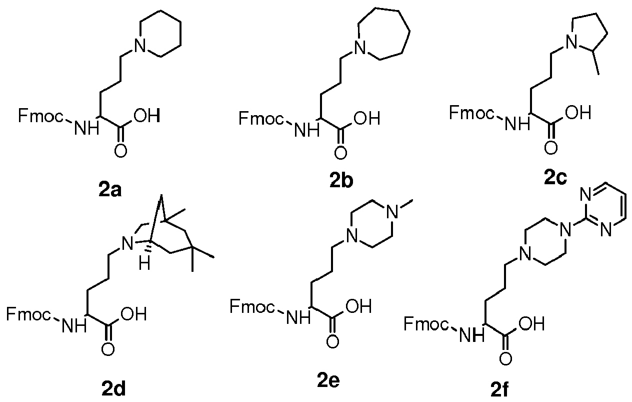

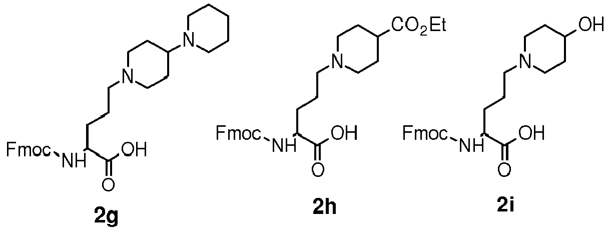

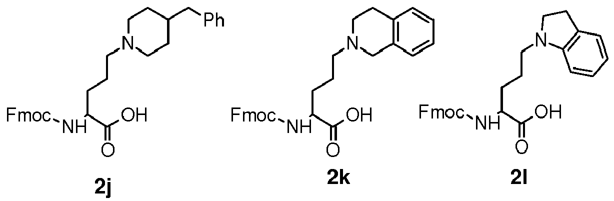

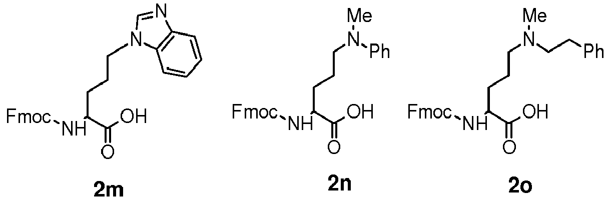

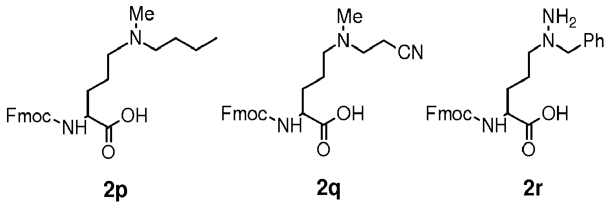

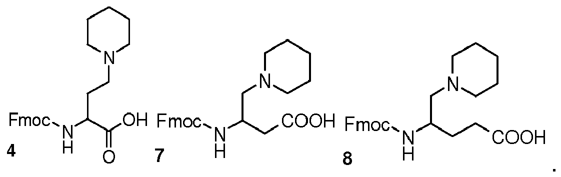

11. Use of the amino acid according to any one of claims 1 to 10, for the preparation of a polypeptide, preferably by liquid or solid phase.

12. A process for the preparation of a compound according to any one of claims 1 to 10, comprising the reaction of a compound of formula (II): 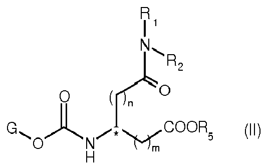 with Ru3(CO)12, in the presence of a solvent, preferably THF, to obtain a compound of formula (III): 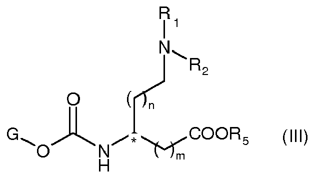 wherein G, R₁, R₂, m and n are defined according to any one of claims 1 to 10 and R₅ is a protective group, preferably a tert-butyl group.

13. The process according to claim 12, further comprising the following steps:
a) purifying said compound of formula (I): 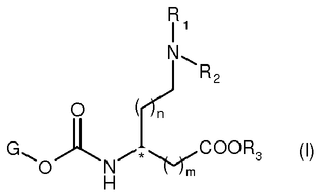 by adding trifluoroacetic acid, while evaporating the solvent, preferably THF, to obtain a compound of formula (IV): 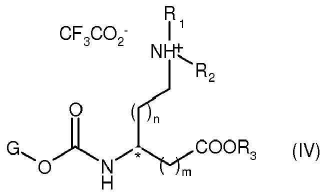 wherein G, R₁, R₂, R₃, m and n are defined according to any one of claims 1 to 10;
b) obtaining a salt or a hydrate salt of the compound of formula (I), preferably by adding an acid, and
c) optionally neutralizing the salt to obtain a compound of formula (I), its hydrates, its polymorphic crystalline structures, racemates, diastereomers or enantiomers.

14. A method for preparing a compound comprising at least one amino acid, wherein said method comprising the following steps:
i) preparing at least one amino acid as defined in any one of claims 1 to 10,
ii) deprotecting said amino acid,
iii) reacting the deprotected amino acid with a carboxylic acid in presence of a coupling reagent, and
iv) obtaining a compound comprising at least one amino acid.

15. The method of claim 14, wherein said compound comprising at least one amino acid is a polypeptide.
